# EUROPÄISCHE PATENTSCHRIFT

(11) **EP 1 360 160 B1**
(45) Veröffentlichungstag und Bekanntmachung des Hinweises auf die Patenterteilung: **11.03.2009**
(21) Anmeldenummer: 01986943.7
(22) Anmeldetag: 28.12.2001
(51) Int. Cl.: C07C 11/02, C07C 2/28

(54) **VERFAHREN ZUR HERSTELLUNG VON HOCHREINEM DIISOBUTEN**
METHOD FOR PRODUCING HIGH-PURITY DIISOBUTENE
PROCEDE DE PRODUCTION DE DIISOBUTENE DE GRANDE PURETE

(30) Priorität: 13.02.2001 DE 10106593; 20.03.2001 DE 10113381
(43) Veröffentlichungstag der Anmeldung: 12.11.2003
(73) Patentinhaber: Evonik Oxeno GmbH, 45772 Marl (DE)
(72) Erfinder: BECKMANN, Andreas, 45657 Recklinghausen (DE); NIERLICH, Franz, 45768 Marl (DE); PETERS, Udo, 45770 Marl (DE); BÜSCHKEN, Wilfried, 45721 Haltern (DE); KERKER, Lothar, 48249 Dülmen (DE)
(74) Vertreter: Hirsch, Hans-Ludwig
(86) Internationale Anmeldenummer: PCT/EP2001/015365
(87) Internationale Veröffentlichungsnummer: WO 2002/064531

(56) Entgegenhaltungen:
- US-A- 4 100 220

## Beschreibung

Die Erfindung betrifft ein Verfahren zur Herstellung von hochreinem Diisobuten aus Isobuten oder Isobuten-haltigen Kohlenwasserstoffgemischen.

Diisobuten, ein Gemisch aus 2,4,4-Trimethylpent-1-en und 2,4,4-Trimethylpent-2-en, wird großtechnisch zu 2,2,4-Trimethylpentan hydriert. Dieser Kohlenwasserstoff ist wegen seiner hohen Octanzahl eine begehrte Vergaserkraftstoffkomponente. Für diesen Zweck können auch Diisobutengemische genutzt werden, die andere C₈-Isomere oder Kohlenwasserstoffe mit anderen C-Zahlen enthalten. Für die Verwendung in Synthesen sind dagegen höhere Reinheiten des Diisobutens nötig. So sind für die Herstellung von 3,5,5-Trimethylhexanal durch Hydroformylierung hochreine Gemische erforderlich. Dieser Aldehyd kann zur entsprechenden Carbonsäure oxidiert werden, die Einsatz zur Herstellung von Peroxiden, Schmiermittel und Sikkativen findet. Weiterhin wird Diisobuten zur Alkylierung von Phenolen eingesetzt. Die dabei entstehenden Alkylaromaten sind Zwischenprodukte für die Herstellung von Detergenzien.

Die Oligomerisierung von Isobuten kann durch Lewis-, Brönstedsäuren oder Koordinationsverbindungen, insbesondere denen des Nickels katalysiert werden. Dabei entstehen Oligomere mit unterschiedlichen Molmassen, da bereits gebildete niedrigere Oligomere (C₈-, C₁₂- Olefine) sich mit Isobuten oder anderen Oligomeren zu höhermolekularen Olefinen umsetzen können. Wenn die Edukte auch n-Butene enthalten, können im Produkt auch Cooligomere vorhanden sein.

Für die wirtschaftliche Herstellung von 2,4,4-Trimethylpentenen (1 und/oder 2) durch Isobutendimerisierung ist daher neben einer guten Raum-Zeit-Ausbeute auch eine hohe C₈-Selektivität und eine hohe Cs-Isomerenreinheit notwendig. Diese Größen können durch die Art des verwendeten Katalysators und die Reaktionsbedingungen beeinflusst werden. Daher darf der eingesetzte Katalysator weder eine Gerüstisomerisierung während der C₈-Bildung, noch eine Isomerisierung des bereits gebildeten C₈-Olefins bewirken. Anderenfalls entsteht ein Produkt, das nur bedingt für chemische Synthesen eingesetzt werden kann.

Die Oligomerisierung kann prinzipell homogen, d. h. unter Verwendung von im Reaktionsgemisch löslichen Katalysatoren, oder heterogen, d.h. unter Verwendung von im Reaktionsgemisch unlöslichen Katalysatoren durchgeführt werden. Der Nachteil der homogenen Verfahren besteht darin, dass der Katalysator den Reaktor mit den Reaktionsprodukten und nicht umgesetzten Edukten verlässt, von denen er abgetrennt, aufgearbeitet und entsorgt oder zurückgeführt werden muss.

Die meisten technischen Verfahren verwenden daher Katalysatoren, die im Festbett angeordnet sind, sodass eine aufwendige Katalysatorabtrennung entfällt. Die meisten bekannten Festkatalysatoren gehören einer der folgenden Gruppen an:
a) Mineralsäuren (z. B. Schwefelsäure oder Phosphorsäure) auf einem Trägermaterial (z. B. Aluminiumoxid oder Siliciumdioxid)
b) Zeolithe oder andere Alumosilikate mit oder ohne weitere Metalle(n), insbesondere mit Übergangsmetallen
c) Saure Ionenaustauscherharze

Für die Herstellung eines hochreinen Gemisches der beiden 2,4,4-Trimethylpentene aus Isobuten sind Mineralsäuren auf Trägern nicht geeignet, da sie auch Gerüstumlagerungen bewirken.

In EP 0 224 220 wird eine Buten-Oligomerisierung an einem mit Wismut und/oder Blei dotierten Zeolith-Katalysator durchgeführt. Dabei enthält die C₈-Fraktion über 4 % unerwünschte 2,3,4-Trimethylpentene.

Die Oligomerisierung von Isobuten an einem Röntgen-amorphen Alumosilikat wird in EP 0 536 839 A2 offengelegt. Dabei kann selbst bei den milden Temperaturen von 60 - 65 °C ein Verlust an 2,2,4-Trimethylpentenen durch Skelettisomerisierung nicht vermieden werden.

Die Isobuten-Oligomerisierung an einem Röntgen-amorphen Nickelalumosilikat wird in WO 93/06926 beschrieben. Dabei wird unverdünntes Isobuten bei 60°C umgesetzt. Das Produktspektrum zeigt, dass die C₈-Selektivität nicht besonders hoch ist. Bei einem Isobuten-Umsatz von 15 - 20% beträgt die C₈-Selektivität 85 - 86%, bei einem Umsatz von 75 % nur noch 72%.

In EP 0 417 407 A1 werden Formkörper von stark sauren Ionenaustauschen als Katalysator für die Oligomerisierung von Isobuten eingesetzt. Diese Ionenaustauscher werden z. T. nachträglich mit Säure behandelt, um eine erhöhte Acidität zu erreichen. Die Ausbeute an Dimeren von 93 - 96 % ist gut. Allerdings wird die Zusammensetzung der C₈-Fraktion nicht offenbart.

Die Verwendung von Moderatoren, wie beispielsweise Methyl-tert.-butylether oder tert.-Butanol, zur Einstellung der Katalysatoraktivität sauerer Ionenaustauscherharze erweist sich vorteilhaft in Hinblick auf das Produktspektrum. Der prinzipielle Nachteil besteht darin, dass der Moderator vom Produkt abgetrennt werden muss und dass es schwierig ist, ein C₈-Olefingemisch ohne Spuren des Moderators zu gewinnen.

In US 44 47 668 wird ein Koppelverfahren beschrieben, bei dem zunächst MTBE in hochreines Isobuten und Methanol an einen sauren Ionenaustauscher gespalten wird. Optional kann das so erhaltene Isobuten in flüssiger Phase in Gegenwart von Methyl-tert.-butylether (MTBE) an einem sauren Ionenaustauscherharz oligomerisiert werden. MTBE dient als Lösungsmittel und zur Steuerung der Katalysatoraktivität. Nach dem Abdestillieren des MTBE verbleibt ein Oligomerisat, das mindestens zu 97 % aus Diisobuten besteht. Weder über den verwendeten Oligomerisationskatalysator, noch über die Isomerenzusammensetzung der C₈-Fraktion werden nähere Angaben gemacht.

US 5 877 372 beschreibt ein Verfahren zur Herstellung von " Isooctan" (hydriertes Diisobuten) aus tert.-Butanol. Dabei ist eine Verfahrensstufe die Oligomerisierung von Isobuten an einem saueren Ionenaustauscherharz. Zur Einstellung der gewünschten Katalysatoraktivität enthält das Edukt für diese Stufe 1 - 30 % tert.-Butanol und zur Erhöhung der C₈-Olefin-Selektivität 30 - 80 % "Isooctan". Das Reaktionsgemisch wird destillativ in ein Kopfprodulct, das tert.-Butanol und nicht umgesetztes Isobuten enthält, und in ein Sumpfprodukt, das aus "Isooctan" und den höheren Oligomeren besteht, getrennt. Der Oligomerenanteil ist zu über 90% Diisobuten. Dieses Gemisch wird zu " Isooctan" mit einigen Prozenten höhermolekularer, gesättigter Kohlenwasserstoffe hydriert, das anteilig in die Dimerisierungsstufe zurückgefahren wird. Als Katalysatoren werden handelsübliche saure Ionenaustauscherharze verwendet.

Es ist ein hoher destillativer Aufwand notwendig, um aus einem nach diesem Verfahren gewonnenen Oligomerisat, die C₈-Olefine abzutrennen; denn die Siedepunkte von "Isooctan" 2,2,4-Trimethylpentan (99 °C) 2, 4,4-Trimethylpent-1-en (100 - 102 °C), und 2,4,4-Trimethylpent-2-en, (102 - 105 °C) liegen nahe beieinander. Darüber hinaus ist nicht bekannt, wie die nach diesem Verfahren hergestellte C₈-Olefinfraktion zusammengesetzt ist.

In GB 2 325 237 A wird ein Verfahren zur Herstellung von einem Di-isobutenhaltigen Gemisch beschrieben, bei dem Isobuten an einem saueren Ionenaustauscherharz in Gegenwart von Methanol und Methyl-tert.-butylether umgesetzt wird. Die Umsetzung erfolgt in zwei hintereinander geschalteten Reaktoren mit Zwischenabtrennung der Produkte nach dem ersten Reaktor. Das Produktgemisch aus beiden Reaktoren besteht nach Abtrennung der Leichtsieder bis zur 90 % aus Dimerisat, höheren Oligomeren und dem vom Dimerisat abgeleiteten Methylether. Auch hier ist das Ziel, eine hochoctanige Komponente bzw. eine Vorstufe für Vergaserkraftstoffe zu gewinnen. Die Gewinnung von hochreinem Diisobuten ist dagegen weder vorgesehen noch beschrieben.

Da die bekannten Verfahren hinsichtlich der C₈-Selektivität und/oder der Reinheit der C₈-Fraktion nicht überzeugen, bestand die Aufgabe, ein besseres Verfahren für die Herstellung eines hochreinen Gemisches der beiden isomeren 2,4,4-Trimethylpentene (1 und/oder 2) zu entwickeln.

In US-A 4,100,220 wird ein Verfahren zur Herstellung von Diisobuten an einem Festen Sulfonsäure - Ionenaustauscherharz beschrieben, bei dem die Säureprotonen nicht durch metallionen ausgetauscht sind.

Es wurde überraschend gefunden, dass bei der Oligomerisierung von Isobuten in flüssiger Phase an einem saurem Ionenaustauscherharz mit Sulfonsäurengruppen die Selektivität der C₈-Olefin-Bildung und der Gehalt der C₈-Fraktion an 2,4,4-Trimethylpentenen erhöht wird, wenn ein Teil der Protonen durch Metallionen ausgetauscht sind.

Die Erfindung betrifft demnach ein Verfahren zur Herstellung von hochreinem Diisobuten, wobei die C8 Fraktion zu über 99,7 Gew.-% aus 2,4,4-Trimethylpentenen besteht, durch Umsetzung von Isobuten oder Isobuten-haltigen Kohlenwasserstoffgemischen an einem festen sauren Ionenaustauscherharz, wobei das saure Ionenaustauscherharz Sulfonsäuregruppen enthält, deren Protonen teilweise gegen Metallionen ausgetauscht wurden.

Saure Ionenaustauscherharze sind für die Oligomerisierung von Isobuten nur dann brauchbare Katalysatoren, wenn sie eine gewisse Mindestacidität aufweisen. So sind Harze mit Carbonsäuregruppen nicht acide genug und daher als Katalysator nicht geeignet. Geeignete Harze sind jene mit Sulfonsäuregruppen. Wie bereits oben erwähnt, entstehen beim Umsatz von Isobuten an sulfonierten Ionenaustauscherharzen Nebenprodukte, wenn nicht ständig ein Regulator mit dem Edukt zugeführt wird.

Aus der Literatur ist bekannt, die Säurestärke von Ionenaustauschern mit Sulfonsäuregruppen durch teilweisen Ionenaustausch herabzusetzen (Structure-breaking Effect of Metal Ions influencing the Acidity of an Anhydrous Acid, C. Buttersack, H. Widdecke, J. Klein, Journal of Molecular Catalysis, '40 (1987) 23 -25). Es war jedoch nicht offenkundig, dass ein derart modifiziertes Ionenaustauscherharz vorteilhaft für die Oligomerisierung von Isobuten eingesetzt werden könnte.

Im erfindungsgemäßen Verfahren werden feste sulfonierte Ionenaustauscherharze eingesetzt, bei denen 30 bis 90 % der Protonen der Sulfonsäuregruppen, vorzugsweise 50 bis 80 %, gegen Metallionen ausgetauscht worden sind. Als Metallionen, die die Protonen ersetzen, können Alkalimetall-, Erdalkalimetall-, Chrom-, Mangan-, Eisen, Kobalt-, Nickel, Zink- und Aluminiumionen sowie Ionen der Lanthanidgruppe verwendet werden. Bevorzugt werden dafür Alkalimetallionen, insbesondere Natriumionen eingesetzt. Es ist auch möglich, dass das Harz mit zwei oder mehreren unterschiedlichen Metallionen beladen ist.

Geeignete Ionenaustauscherharze sind beispielsweise solche, die durch Sulfonierung von Phenol/Aldehyd-Kondensaten oder von Cooligomeren von aromatischen Vinylverbindungen hergestellt werden. Beispiele für aromatische Vinylverbindungen zur Herstellung der Cooligomere sind: Styrol, Vinyltoluol, Vinylnaphthalin, Vinylethylbenzol, Methylstyrol, Vinylchlorbenzol, Vinylxylol und Divinylbenzol. Insbesondere werden die Cooligomeren, die durch Umsetzung von Styrol mit Divinylbenzol entstehen, als Vorstufe für die Herstellung von Ionenaustauscherharzen mit Sulfongruppen verwendet. Die Harze können gelförmig, makroporös oder schwammförmig hergestellt werden. Stark saure Harze des Styrol-Divinyl-Typs werden u. a. unter folgenden Handelsnamen verkauft: Duolite C20, Duolite C26, Amberlyst 15, Amberlite IR-120, Amberlite 200, Dowex 50, K2611, K 2431.

Die Eigenschaften dieser Harze, insbesondere spezifische Oberfläche, Porosität, Stabilität, Quellung bzw. Schrumpfung und Austauschkapazität, können durch den Herstellprozess variiert werden.

Die Ionenaustauscherkapazität liegt zwischen 1 bis 2, insbesondere 1,5 bis 1,9 Mol H⁺ pro Liter feuchtes Harz (handelsüblich).

Im Verfahren der Erfindung werden vorzugsweise macroporöse Harze eingesetzt, wie beispielsweise K 2431. Das Porenvolumen beträgt 30 bis 60 ml/g , insbesondere 40 bis 50 ml/g (bezogen auf handelsübliches wasserfeuchtes Harz).

Die Korngröße des Harzes liegt zwischen 500 µm und 1500 µm, insbesondere zwischen 600 µm und 1000 µm.

Die Korngrößenverteilung kann enger oder weiter gewählt werden. So können beispielsweise Ionenaustauscherharze mit sehr einheitlicher Korngröße (monodisperse Harze) eingesetzt werden.

Bei der Verwendung mehrerer Reaktoren können diese mit Harz gleicher oder unterschiedlicher Korngröße (bzw. Korngrößenverteilung) gefüllt sein.

Es kann vorteilhaft sein, in Reaktoren, die mit hohen Lineargeschwindigkeiten durchströmt werden, zur Verringerung des Differenzdruckes ein größeres Korn, und in Reaktoren, die mit einer geringen Lineargeschwindigkeit durchströmt werden, zur Erzielung des optimalen Umsatzes ein kleineres Korn einzusetzen.

Optional können die Ionenaustauscherharze als Formkörper, wie beispielsweise Zylinder, Ringe oder Kugeln, eingesetzt werden.

Für die Herstellung der Katalysatoren mit den gewünschten Aktivitäten gibt es mehrere Möglichkeiten. Liegt das Ionenaustauscherharz in der H-Form vor, können Protonen gegen Metallionen ausgetauscht werden. Liegt das Harz als Metallsalz vor, können Metallionen mit Hilfe von Säuren durch Protonen ersetzt werden. Prinzipiell kann dieser Ionenaustausch sowohl in organischer als in wässriger Suspension erfolgen. Dabei wird das Iönenauscherharz mit so viel Flüssigkeit aufgeschlämmt, dass eine gut rührbare Suspension entsteht. Dazu wird eine Lösung, die die gewünschten Ionen enthält, zudosiert. Nach erfolgten Ionenaustausch wird das teilausgetauschte Ionenaustauscherharz gewaschen und getrocknet.

Ein bevorzugter Weg zur Herstellung der im erfindungsgemäßen Verfahren verwendeten Katalysatoren ist der Austausch von Protonen gegen Metallionen in wässriger Phase.

Bei der Herstellung des Katalysators wird das Ionenaustauscherharz im Einfachen bis zum Zehnfachen, insbesondere im Einfachen bis zum Dreifachen seines Volumens in einem Lösungsmittel suspendiert.

Für die Herstellung der Lösung mit der gewünschten Ionenart, die zudosiert wird, ist es ratsam, ein Lösungsmittel zu wählen, das mit dem Lösungsmittel, in dem das Harz suspendiert ist, mischbar ist. Es ist zweckmäßig, gleiche Lösungsmittel zu verwenden.

Die Ionen, mit denen das Harz beladen werden soll, können als Lösungen von Säuren, Hydroxiden, oder Salzen organischer oder anorganischer Säuren vorliegen. Bei Salzen mehrbasiger Säuren können auch saure Salze eingesetzt werden. Ebenfalls können Verbindungen mit anderen organischen Resten wie beispielsweise Alkoholate oder Acetylacetonate verwendet werden.

Der Ionenaustausch erfolgt unter Rühren im Temperaturbereich 10 bis 100 °C, insbesondere 20 bis 40 °C.

Die Zutropfzeit der Ionenlösung beträgt 0,5 bis 12 h, insbesondere 1 bis 3 h.

Die Austauschzeit (von Beginn des Zutropfens an) liegt zwischen 1 und 24 h, insbesondere zwischen 3 und 12 h.

Nach dem Ionenaustausch wird der Katalysator von der Lösung getrennt, z. B. durch Dekantieren oder Filtrieren, und optional anschließend mit einem Lösungsmittel gewaschen. Es ist zweckmäßig das gleiche Lösungsmittel zu verwenden, in dem der Katalysator suspendiert war.

Der feuchte Katalysator wird getrocknet, einerseits um ihn handhabbarer (rieselfähiger) zu machen und um andererseits in den ersten Tagen nach dem Anfahren des Reaktors die Verunreinigung des Produktes durch das anhaftende Lösungsmittel oder dessen Folgeprodukte gering zu halten. Die Trocknung kann im Vakuum oder im Inertgasstrom, beispielsweise im Stickstoffstrom, erfolgen. Die Trockentemperaturen liegen zwischen 10 und 120 °C, insbesondere zwischen 40 und 80 °C. Je nach Druck und Temperatur betragen die Trocknungszeiten 1 bis 24 h.

Nach dem oben beschriebenen Vorgehen können je nach Austauschgrad, Ionenart und Harz Katalysatoren unterschiedlicher Aktivität hergestellt werden.

Ein Reaktor kann ein Gemisch von Harzen unterschiedlicher Reaktivität enthalten. Ebenso ist es möglich, dass ein Reaktor Katalysatoren mit unterschiedlicher Aktivität, in Schichten angeordnet, enthält. Wird mehr als ein Reaktor verwendet, können die einzelnen Reaktoren mit Katalysatoren gleicher oder unterschiedlicher Aktivität(en) gefüllt sein.

Als Ausgangsstoff kann reines Isobuten oder ein isobutenhaltiges Kohlenwasserstoffgemisch, das keine weiteren ungesättigten Verbindungen enthält, eingesetzt werden. Bei Verwendung von reinem Isobuten ist es ratsam, zur Herabsetzung der Konzentration ein leicht abtrennbares Lösungsmittel zuzusetzen.

Technische Gemische, die Isobuten enthalten, sind beispielsweise Leichtbenzinfraktionen aus Raffinerien, C₄-Fraktionen aus FC- oder Steamcracker, Gemische aus Fischer-Tropsch-Synthesen, Gemische aus Dehydrierung von Butanen, Gemische aus Skelettisomerisierung linearer Butene, Gemische, entstanden durch Metathese von Olefinen oder anderen technischen Prozessen.

Aus diesen technischen Gemischen muss vor der erfindungsgemäßen Verwendung Isobuten von den anderen ungesättigten Verbindungen abgetrennt werden. Die Gewinnung von Isobuten aus dem C₄-Schnitt eines Steamcrackers wird weltweit angewandt und sei hier beispielsweise beschrieben. Die Abtrennung des Isobutens umfasst dabei im Wesentlichen folgende Verfahrensstufen: Der erste Schritt ist die Entfernung des größten Teils des Butadiens. Kann Butadien gut vermarktet werden oder besteht ein Eigenverbrauch, wird es durch Extraktion abgetrennt. Im anderen Fall wird es bis zu einer Restkonzentration von ca. 2000 ppm selektiv zu linearen Butenen hydriert, wie z. B. beschrieben in EP 52 3482. Zurück bleibt in beiden Fällen ein Kohlenwasserstoffgemisch (Raffinat I oder hydriertes Crack-C4), das neben den gesättigten Kohlenwasserstoffen, n- Butan und Isobutan, die Olefine Isobuten, 1-Buten und 2-Butene enthält. Aus diesem Gemisch kann Isobuten durch Umsetzung mit Methanol zu Methyl-tert.-butylether (MTBE) oder mit Wasser zu tert.-Butanol (TBA) abgetrennt werden. Sowohl MTBE als auch TBA können in Umkehrung ihrer Bildung in Isobuten und Methanol oder Wasser zurückgespaltet werden, so z. B. beschrieben in DE 100 20 943.

Optional kann Raffinat I, hydriertes Crack-C₄ oder ein ähnlich zusammengesetztes Kohlenwasserstoffgemisch in einer Reaktivkolonne hydroisomerisiert werden. Dabei kann ein Gemisch aus Isobuten und Isobutan als Kopfprodukt gewonnen werden.

Das Verfahren gemäß der Erfindung kann in chargenweise oder bevorzugt kontinuierlich arbeitenden Reaktoren, die üblicherweise bei Feststoff/Flüssigkeits-Kontaktreaktionen zum Einsatz gelangen, durchgeführt werden. Bei der Verwendung von kontinuierlich arbeitenden Strömungsreaktoren bedient man sich meistens, jedoch nicht ausschließlich, eines Festbetts. Wenn ein Festbett-Strömungsreaktor verwendet wird, kann die Flüssigkeit aufwärts oder abwärts strömen. Meistens wird ein Abwärtsströmen der Flüssigkeit bevorzugt. Weiterhin ist es möglich, den Reaktor unter Produktrückführung oder im geraden Durchgang zu betreiben.

Bei der Verwendung von Rohrreaktoren kann das Verhältnis von Länge zu Durchmesser der Katalysatorschüttung variiert werden, entweder durch die geometrischen Maße des Reaktors oder durch dessen Füllgrad. Bei gleicher Kontaktmenge und Belastung (LHSV) können somit unterschiedliche Leerrohrgeschwindigkeiten erreicht werden. Reaktoren, bei denen ein Teil des Reaktionsgemisches, bevorzugt nach Abtrennung der Oligomeren, zurückgeführt wird, können mit Leerrohrgeschwindigkeiten von 1 bis 30 m/s, insbesondere mit 2 bis 20 m/s, ganz besonders mit 4 bis 10 m/s betrieben werden. In den Reaktoren, die im geraden Durchgang durchströmt werden, können die Leerrohrgeschwindigkeiten im Bereich von 0,1 bis 20 m/s, insbesondere im Bereich von 0,8 bis 8 m/s, liegen.

Aufgrund der durch Ionenaustausch herabgesetzten Katalysatoraktivität sind geringere Geschwindigkeiten als bei einem nicht ausgetauschten Ionenaustauscherharz möglich, da wegen der geringeren Aktivität Temperaturspitzen besser vermieden werden können.

Die Katalysatorbelastung (LHSV) beträgt bei Reaktoren, die unter Produktrückführung, bevorzugt nach Abtrennung der Produkte, betrieben werden, 0,5 bis 15 h⁻¹, insbesondere 1 bis 10 h⁻¹, ganz besonders 2 bis 5 h⁻¹. Bei Reaktoren, die im geraden Durchgang durchströmt werden, liegen die Belastungen im Bereich von 1 bis 50 h⁻¹, insbesondere bei 5 bis 30 h⁻¹.

Die Anzahl der im erfindungsgemäßen Verfahren in Reihe geschalteten Reaktoren liegt zwischen 1 und 10, bevorzugt zwischen 1 und 4.

Eine bevorzugte Verfahrensvariante ist, den ersten Reaktor unter Produktrückführung und die folgenden Reaktoren im geraden Durchgang zu betreiben.

Jeder Reaktor kann adiabatisch, polytrop oder praktisch isotherm betrieben werden. Praktisch isotherm bedeutet, daß die Temperatur an einer beliebigen Stelle im Reaktor maximal um 10°C höher ist als die Temperatur am Reaktoreingang.

Die Temperaturen, bei denen die Reaktoren betrieben werden, liegen zwischen 20 und 120°C, vorzugsweise zwischen 40 und 100°C. Die Temperatur ist abhängig von der Aktivität des Katalysators (u. a. Austauschgrad).

Die erfindungsgemäße Umsetzung kann bei einem Druck gleich oder über dem Dampfdruck des Einsatz-Kohlenwasserstoffgemisches bei der jeweiligen Reaktionstemperatur durchgeführt werden, vorzugsweise bei einem Druck unter 40 bar. Um in den Reaktoren Verdampfungsprobleme zu vermeiden, sollte der Druck 2 bis 4 bar höher als der Dampfdruck des Reaktionsgemisches sein.

Der Gesamtumsatz an Isobuten hängt von der Art und Menge des verwendeten Katalysators, den eingestellten Reaktionsbedingungen und Anzahl der Reaktionsstufen ab. Aus wirtschaftlichen Gründen wird der Isobuten-Umsatz im Bereich von 50 bis 100 %, vorzugsweise zwischen 90 und 100 % gehalten. Darüber hinaus ist es sinnvoll, um eine hohe Raum-Zeit-Ausbeute und eine hohe C₈-Olefinselektivität zu erhalten, Kohlenwasserstoffe mit einem Isobutengehalt nicht unter 5 %, vorzugsweise nicht unter 10 % einzusetzen.

Um eine möglichst hohe Selektivität der C₈-Olefinbildung zu erreichen, ist es ratsam, die Konzentration an Oligomeren, insbesondere an C₈-Olefine, in jedem Reaktor zu begrenzen. Deren Konzentration liegt im Bereich 0 bis 50 %, insbesondere im Bereich von 0 bis 30 %. Die Begrenzung der Konzentration an Oligomeren kann durch Wahl der Betriebsparameter, wie Temperatur oder Verweilzeit, erfolgen. Eine weitere Möglichkeit besteht darin, die Konzentration an Isobuten am Reaktoreingang durch Zusatz eines Verdünnungsmittels unter 50 %, insbesondere unter 30 % zu halten. Zweckmäßig wird ein Verdünnungsmittel verwendet, das im Edukt vorhanden ist und nach teilweiser oder praktisch vollständiger Umsetzung des Isobutens aus dem Reaktionsgemisch zurückgewonnen wird. Eine bevorzugte Ausführung des Verfahrens ist es daher, das Reaktoraustrittsgemisch in eine Fraktion, die die Oligomeren enthält, und in eine zweite, die das (die) Verdünnungsmittel und gegebenenfalls nicht umgesetztes Isobuten enthält, zu trennen. Das Verdünnungsmittel mit dem darin enthaltenden Isobuten wird zu einem Teil in den gleichen Reaktor oder einen davor geschalteten zurückgeführt und der andere Teil und in einem nachgeschalteten Reaktor geleitet oder aufgearbeitet. Bei mehreren hintereinandergeschalteten Reaktoren kann auch mehr als eine Oligomerenabtrennung vorhanden sein.

Die abgetrennten Oligomere (aus einer oder mehreren Trenneinheiten) werden in einer weiteren Destillation in C₈-Olefine, C₁₂-Olefine und höhere Oligomere getrennt.

Optional kann die Oligomerisierung in einer Reaktivdestillationskolonne, die das ausgetauschte Katalysatorharz enthält, durchgeführt werden. Dabei gelten die oben genannten Temperatur- und Druckbereiche. Als Sumpfprodukt fällt dabei das Oligomerengemisch an. Das Kopfprodukt besteht aus Lösungsmittel und gegebenenfalls nicht umgesetztes Isobuten. Die Aufarbeitung dieser Ströme erfolgt wie oben beschrieben.

Weiterhin ist es möglich, das erfindungsgemäße Verfahren in einer Anlage zu betreiben, die aus einem oder mehreren Reaktor(en) und einer Reaktivdestillationskolonne besteht.

Das nach dem erfindungsgemäßen Verfahren hergestellte hochreine Gemisch der beiden 2,4,4-Trimethylpenten-Isomere kann zu 3,5,5-Trimethylhexanal hydroformyliert werden. Dieser Aldehyd kann zur entsprechenden Carbonsäure oxidiert oder zum entsprechenden Alkohol hydriert werden. Die 3,5,5-Trimethylhexansäure findet Einsatz zur Herstellung von Peroxiden, Schmiermittel und Sikkativen. Weiterhin wird Diisobuten zur Alkylierung von Phenol oder Phenolderivaten eingesetzt. Die dabei entstehenden Alkylaromaten sind Zwischenprodukte für die Herstellung von Detergenzien. Darüber hinaus wird Diisobuten zur Alkylierung aromatischer Amine eingesetzt.

Das erfindungsgemäße Verfahren hat folgende Vorteile:
- Bei praktisch 100 %igem Umsatz beträgt die Ausbeute an 2,4,4-Trimethylpentenen 80 bis 85 %. Weiterhin entstehen 15 bis 20 % höhere Oligomere, davon ca. 10 % C₁₂-Olefine. Die C₈-Fraktion besteht zu über 99,7 %, meistens zu über 99,8 % aus 2,4,4-Trimethylpentenen und kann aufgrund ihrer hohen Reinheit als Ausgangsstoff für chemische Synthesen verwendet werden.
- Zur Steuerung der Produktqualität ist es nicht notwendig, einen Hilfsstoff zuzusetzen, was die Prozessführung vereinfacht.
- Da keine Hilfsstoffe zugesetzt werden, brauchen diese bzw. ihre Folgeprodukte nicht aus dem Reaktionsgemisch, insbesondere von den Zielprodukten, abgetrennt werden. Dies bedeutet einerseits keine Kosten für die Hilfsstoffe und andererseits einen geringeren Trennaufwand. Da das erfindungsgemäß hergestellte Produkt keine Spuren eines Fremdstoffes (Moderator) enthält, werden Folgereaktionen nicht beeinträchtigt.

Die folgenden Beispiele sollen die Erfindung weiter erläutern, nicht aber ihren Anwendungsbereich beschränken, der sich aus den Patentansprüchen ergibt.

Die Herstellung der teilneutralisierten Ionenaustauscherharze erfolgte, indem das saure Ionenaustauscherharz, suspendiert in Wasser, mit der berechneten Menge einer wässrigen Alkalihydroxid-Lösung umgesetzt wird, wie exemplarisch im Beispiel 1 beschrieben.

### Beispiel 1

### Herstellung eines teilneutralisierten Katalysators, Einstellung der Säurekapazität

Der eingesetzte Ionenaustauscher der Fa. Rohm und Haas (Amberlyst 15) hatte eine ursprüngliche Säurekapazität von 1,43 mol In+/1. Zur Einstellung der gewünschten Aktivität wurden 50 % der sauren Zentren neutralisiert.

Hierfür wurden 1000 ml des Ionenaustauscherharzes in 1000 ml VE-Wasser aufgeschlämmt und unter starkem Rühren eine Lösung aus 28,6g Natriumhydroxid (0,715 mol) und 500 ml VE-Wasser in einer Stunde im Temperaturbereich 20 bis 40 °C zugetropft. Es wurde 5 min nachgerührt und danach das Ionenaustauscherharz mit dreimal 1000 ml VE-Wasser neutral gewaschen. Die anschließende Kapazitätsmessung des teilneutralisierten Ionenaustauschers ergab 0,715 +/- 0,03 mol H+/1. Der Katalysator wurde vor 15 h bei 70 °C getrocknet.

Die Oligomerisierungsversuche (Beispiele 2 bis 5) wurden in einem ummantelten Laborrohrreaktor von 2 m Länge und 2 cm inneren Durchmesser durchgeführt. Mit Hilfe eines Wärmeträgeröls, das durch den Reaktormantel gepumpt wurde, konnte der Reaktor temperiert werden. Bei allen Versuchen wurde ein Isobutan-Isobuten-Gemisch bei 23 bar oligomerisiert.

### Beispiel 2

### Oligomerisierung mit nicht teilneutralisiertem Katalysator (Vergleichsbeispiel)

| Katalysator: | Amberlyst 15 |
|---|---|
| Mantel-Temp. (°C): | 40 |
| Einsatzgemisch | |
| Isobutan (Gew.-%) | 83 |
| Isobuten (Gew.-%) | 16 |
| n-Butan (Gew.-%) | 1 |
| LHSV (h-1) 13 | |
| Umsatz Isobuten (%) | 99,7 |
| Selektivitäten (%) | |
| Di-Isobuten | 19 |
| Tri-Isobuten | 61 |
| hohere Oligomere | 19 |
| 2,4,4-Trimethypentene im C₈ (Gew.-%) | 67 |

Aufgrund der hohen Säurestärke und -kapazität ist der Umsatz sehr hoch und die Selektivität zum Dimeren sehr schlecht. Die gebildeten C₈-Olefine bestehen wegen Umalkylierung während oder nach ihrer Bildung nur zum Teil aus den gewünschten Trimethylpentenen

### Beispiel 3

### Oligomerisierung mit teilneutralisiertem Katalysator (erfindungsgemäß)

| Katalysator: | Amberlyst 15; 50 % H+ gegen Na+ ausgetauscht |
|---|---|
| Mantel-Temp (°C): | 40 |
| Einsatzgemisch | |
| Isobutan (Gew.-%) | 84 |
| Isobuten (Gew.-%) | 15 |
| n-Butan (Gew.-%) | 1 |
| LHSV (h-1) | 13 |
| Umsatz Isobuten (%) | 78 |
| Selektivitäten (%) | |
| Di-Isobuten | 63 |
| Tri-Isobuten | 33 |
| hohere Oligomere | 3 |
| 2,4,4-Trimethypentene im C₈ (Gew.-%) | >99,9 |

Der Vergleich mit Beispiel 2 zeigt, dass die Teilneutralisation zwar eine Reduzierung des Umsatzes, jedoch eine Verbesserung der Dimerenselektivität bei vergleichbaren Reaktionsbedingungen bewirkt.

Das Diisobuten wird in hoher Isomerenreinheit gebildet.

### Beispiel 4

### Oligomerisation mit teilneutralisiertem Katalysator (erfindungsgemäß)

| | |
|---|---|
| Katalysator: | Amberlyst 15; 80 % H+ gegen Na+ ausgetauscht |
| Mantel-Temp (°C): | 110 |
| Einsatzgemisch | |
| Isobutan (Gew.-%) | 0 |
| Isobuten (Gew.-%) | 100 |
| LHSV (h-1) | 4 |
| Umsatz Isobuten (%) | 67 |
| Selektivitäten (%) | |
| Di-Isobuten | 82 |
| Tri-Isobuten | 17 |
| höhere Oligomeres | <1 |
| 2,4,4-Trimethypentene im C₈ (Gew-%) | >99,9 |

Ein 80%iger Austausch der Protonen gegen Natriumionen bewirkt eine deutliche Verringerung der Reaktionsgeschwindigkeit, sodass schärfere Bedingungen (höhere Temperatur und/oder höhere Isobutenkonzentration) für eine technisch vernünftige Reaktionsgeschwindigkeit notwendig sind. Jedoch ergibt sich im Vergleich zum Beispiel 3 trotz der höheren Temperatur eine bessere Dimerenselektivität.

### Beispiel 5 (gemäß der Erfindung)

| Katalysator: | Amberlyst 15; 50 % H+ gegen K+ ausgetauscht |
|---|---|
| Mantel-Temp (°C): | 100 |
| Einsatzgemisch | |
| Isobutan (Gew.-%) | 45 |
| Isobuten (Gew.-%) | 54 |
| n-Butan (Gew.-%) | 1 |
| LHSV (h-1) | 2 |
| Umsatz Isobuten (%) | 13 |
| Selektivitäten (%) | |
| Di-Isobuten | 94 |
| Tri-Isobuten | 5 |
| hohere Oligomere (Gew-%) | <1 |
| 2,4,4-Trimethypentene im C₈ (Gew.-%) | >99,9 |

Dieses Beispiel zeigt, dass es bei einer Verschärfung der Reaktionsbedingungen auch bei Teilneutralisation mit K+ gegen H+ gelingt, Isobuten zu dimerisieren.

Gegenüber den übrigen Beispielen ergibt sich eine nochmals sehr stark verbesserte Dimerenselektivität.

## Patentansprüche

1. Verfahren zur Herstellung von hochreinem Diisobuten, wobei die C₈-Fraktion zu über 99,7 Gew.-% aus 2,4,4-Trimethylpentenen besteht, durch Umsetzung von Isobuten oder Isobuten-haltigen Kohlenwasserstoffgemischen an einem festen sauren Ionenaustauscherharz, **dadurch gekennzeichnet,**
**dass** das saure Ionenaustauscherharz Sulfonsäuregruppen enthält, deren Protonen teilweise gegen Metallionen ausgetauscht wurden.

2. Verfahren nach Anspruch 1,
**dadurch gekennzeichnet,**
**dass** 30 bis 90 % der Protonen gegen Metallionen ausgetauscht sind.

3. Verfahren nach Anspruch 1 oder 2,
**dadurch gekennzeichnet,**
**dass** 50 bis 80 % der Protonen gegen Metallionen ausgetauscht sind.

4. Verfahren nach einem der Ansprüche 1 bis 3,
**dadurch gekennzeichnet,**
**dass** die Metallionen Alkalimetallionen sind.

5. Verfahren nach einem der Ansprüche 1 bis 4,
**dadurch gekennzeichnet,**
**dass** Metallionen Natriumionen sind.

6. Verfahren nach einem der Ansprüche 1 bis 5,
**dadurch gekennzeichnet,**
**dass** die Umsetzung bei 20 bis 120°C erfolgt.

7. Verfahren nach Anspruch 6,
**dadurch gekennzeichnet,**
**dass** die Umsetzung bei 40 bis 100 °C erfolgt.

8. Verfahren nach einem der Ansprüche 1 bis 7,
**dadurch gekennzeichnet,**
**dass** die Umsetzung in flüssiger Phase im Druckbereich von 5 bis 40 bar erfolgt.

## Claims

1. Process for preparing high-purity diisobutene, where the C₈ fraction comprises over 99.7% by weight of 2,4,4-trimethylpentenes, by reaction of isobutene or isobutene-containing hydrocarbon mixtures over a solid acidic ion-exchange resin, **characterized in that** the acidic ion-exchange resin contains sulfonic acid groups whose protons have been partly replaced by metal ions.

2. Process according to Claim 1, **characterized in that** from 30 to 90% of the protons have been replaced by metal ions.

3. Process according to Claim 1 or 2, **characterized in that** from 50 to 80% of the protons have been replaced by metal ions.

4. Process according to any of Claims 1 to 3, **characterized in that** the metal ions are alkali metal ions.

5. Process according to any of Claims 1 to 4, **characterized in that** the metal ions are sodium ions.

6. Process according to any of Claims 1 to 5, **characterized in that** the reaction is carried out at from 20 to 120°C.

7. Process according to Claim 6, **characterized in that** the reaction is carried out at from 40 to 100°C.

8. Process according to any of Claims 1 to 7, **characterized in that** the reaction is carried out in a liquid phase in the pressure range from 5 to 40 bar.

## Revendications

1. Procédé pour la préparation de diisobutène de haute pureté, où la fraction en C₈ est constituée à raison de plus de 99,7% en poids par des 2,4,4-triméthylpentènes, par transformation d'isobutène ou de mélanges d'hydrocarbures contenant de l'isobutène sur une résine échangeuse d'ions acide solide, **caractérisé en ce que** la résine échangeuse d'ions acide contient des groupes acide sulfonique dont les protons ont été partiellement remplacés par des ions métalliques.

2. Procédé selon la revendication 1, **caractérisé en ce que** 30 à 90% des protons sont remplacés par des ions métalliques.

3. Procédé selon la revendication 1 ou 2, **caractérisé en ce que** 50 à 80% des protons sont remplacés par des ions métalliques.

4. Procédé selon l'une quelconque des revendications 1 à 3, **caractérisé en ce que** les ions métalliques sont des ions de métal alcalin.

5. Procédé selon l'une quelconque des revendications 1 à 4, **caractérisé en ce que** les ions métalliques sont des ions de sodium.

6. Procédé selon l'une quelconque des revendications 1 à 5, **caractérisé en ce que** la transformation est réalisée à 20 jusqu'à 120°C.

7. Procédé selon la revendication 6, **caractérisé en ce que** la transformation est réalisée à 40 jusqu'à 100°C.

8. Procédé selon l'une quelconque des revendications 1 à 7, **caractérisé en ce que** la transformation est réalisée en phase liquide dans une plage de pression de 5 à 40 bars.
